(19)

Europäisches
Patentamt
European
Patent Office
Office européen
des brevets

(11) **EP 4 455 198 A1**

(12) **EUROPEAN PATENT APPLICATION**
published in accordance with Art. 153(4) EPC

(43) Date of publication:
**30.10.2024 Bulletin 2024/44**

(21) Application number: **22910646.3**

(22) Date of filing: **09.11.2022**

(51) International Patent Classification (IPC):
*C08J 9/26* (2006.01)        *A61K 8/02* (2006.01)
*A61K 8/73* (2006.01)        *A61K 8/85* (2006.01)
*A61Q 1/00* (2006.01)        *C08J 3/12* (2006.01)
*C08K 5/103* (2006.01)        *C08L 1/10* (2006.01)
*C08L 1/12* (2006.01)        *C08L 3/02* (2006.01)
*C08L 29/04* (2006.01)        *C08L 67/00* (2006.01)
*C08L 67/04* (2006.01)

(52) Cooperative Patent Classification (CPC):
**A61K 8/02; A61K 8/73; A61K 8/85; A61Q 1/00;
C08J 3/12; C08J 9/26; C08K 5/103; C08L 1/10;
C08L 1/12; C08L 3/02; C08L 29/04; C08L 67/00;
C08L 67/04**

(86) International application number:
**PCT/JP2022/041764**

(87) International publication number:
**WO 2023/119927 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.12.2021 JP 2021207238**

(71) Applicant: **Daicel Corporation**
**Osaka-shi, Osaka 530-0011 (JP)**

(72) Inventors:
• **KOBAYASHI, Keiko**
  **Tokyo 108-8230 (JP)**
• **SAKAMOTO, Yuta**
  **Tokyo 108-8230 (JP)**

(74) Representative: **Grünecker Patent- und
Rechtsanwälte
PartG mbB
Leopoldstraße 4
80802 München (DE)**

(54) **POROUS PARTICLE, COSMETIC COMPOSITION, AND PRODUCTION METHOD FOR POROUS
PARTICLE**

(57)    Porous particles contain a biodegradable polymer as a main component. The biodegradable polymer is an aliphatic polyester and/or a polysaccharide ester having a total degree of substitution of 0.7 or more and 3.0 or less. The porous particles have an average particle size of 0.08 $\mu$m or more and 100 $\mu$m or less, a sphericity of 0.7 or more and 1.0 or less, and a relative specific surface area of more than 3.0 and 20 or less. A cosmetic composition contains the porous particles.

FIG. 1

EP 4 455 198 A1

**Description**

Technical Field

The present disclosure relates to porous particles, a production method therefor, and a cosmetic composition containing the porous particles.

Background Art

Typically, various fine polymer particles are blended in cosmetics for purposes such as improving the spread of the cosmetic, changing the texture, imparting a wrinkle blurring effect, and improving the lubricity of a foundation or the like. A light-scattering (soft-focus) effect can be obtained depending on the shape and physical properties of the fine particles. In particular, fine particles having high sphericity are excellent in texture. When used in a foundation or the like, such fine particles fill and smooth out the roughness of the skin, and scatter light in various directions, and thus provide an effect of making wrinkles less noticeable (soft-focus). Porous fine particles have such an advantage that they are excellent in absorption of oil such as sebum and can be used as a scrubbing agent depending on the shape thereof.
Examples of the material of such fine particles that are blended into cosmetics include synthetic polymers, such as polyamide, polymethyl methacrylate, polystyrene, polypropylene, and polyethylene. However, in recent years, due to the problem of marine pollution caused by mircoplastics, there has been a demand for porous fine particles formed from a biodegradable material having necessary properties and causing less environmental load instead of the aforementioned synthetic polymers.
For example, Japanese Patent No. 6543920 (Patent Document 1) discloses polymer fine particles that are formed from an aliphatic polyester having a reduced environmental load and have a linseed oil absorption amount of 120 ml/100 g or more and 1000 ml/100 g or less.

Citation List

Patent Document

Patent Document 1: JP 6543920 B

Summary of Invention

Technical Problem

Patent Document 1 describes, as a method of easily producing porous aliphatic polyester resin fine particles, "a method in which an emulsion is formed in a system where a polymer A, a polymer B, and an organic solvent are dissolved and mixed, and the mixture is subjected to phase separation into two phases; i.e., a solution phase containing the polymer A as a main component and a solution phase containing the polymer B as a main component, and then a poor solvent of the polymer A is brought into contact with the emulsion to precipitate fine particles of the polymer A". However, this method of Patent Document 1 is a method of producing fine particles using a so-called droplet method. In this production method, since the solid content concentration of the solution is low, volume shrinkage occurs after precipitation of particles. Thus, fine particles having high sphericity cannot be produced, and spaces (pores) are less likely to be provided inside the fine particles.
Porous particles formed of a biodegradable polymer material and having excellent physical properties such as texture have not yet been proposed. An object of the present disclosure is to provide porous particles having excellent biodegradability, texture, and soft-focus properties, and a method for producing the porous particles.

Solution to Problem

The porous particles according to an embodiment of the present disclosure contain a biodegradable polymer as a main component. The biodegradable polymer may be an aliphatic polyester, a polysaccharide ester having a total degree of substitution of 0.7 or more and 3.0 or less, or a mixture thereof. The porous particles have an average particle size of 0.08 $\mu$m or more and 100 $\mu$m or less, a sphericity of 0.7 or more and 1.0 or less, and a relative specific surface area of more than 3.0 and 20 or less. The porous particles may have a degree of surface smoothness of 10% or more and 95% or less.
Preferably, the porous particles further contain a plasticizer for the biodegradable polymer. This plasticizer may be selected from a glycerin-based plasticizer and a polycarboxylic acid ester-based plasticizer.

The aliphatic polyester is preferably a polyhydroxyalkanoate, or a polymer of an aliphatic dicarboxylic acid and an aliphatic diol. The aliphatic polyester may be one type or two or more types selected from the group consisting of polycaprolactone, polyhydroxybutyric acid, and polylactic acid. The polysaccharide ester may be a cellulose acylate having an acyl group having 3 or more carbons.

A cosmetic composition according to an embodiment of the present disclosure contains any type of the porous particles described above.

A method for producing porous particles according to an embodiment of the present disclosure includes:

(1) preparing a mixture by mixing a biodegradable polymer, a plasticizer, a first thermoplastic polymer, and a second thermoplastic polymer different from the first thermoplastic polymer;
(2) melt-kneading the mixture at 200°C or higher and 280°C or lower to yield a kneaded product; and
(3) removing the first thermoplastic polymer and the second thermoplastic polymer from the kneaded product.

When an SP value of the biodegradable polymer is SPa, an SP value of the first thermoplastic polymer is SPb, and an SP value of the second thermoplastic polymer is SPc, it is preferable that SPa, SPb, and SPc satisfy the following relational expression:

$$0.05 \leq |SPc - SPa|/|SPb - SPa| \leq 1.5.$$

The first thermoplastic polymer may be selected from the group consisting of polyvinyl alcohol and thermoplastic starch. The second thermoplastic polymer may be polyethylene glycol. The plasticizer may be selected from the group consisting of a glycerin-based plasticizer and a polycarboxylic acid ester-based plasticizer.

The blended amount of the plasticizer may be more than 0 parts by weight and 120 parts by weight or less relative to 100 parts by weight of the biodegradable polymer. The blended amount of the first thermoplastic polymer may be 110 parts by weight or more and 15000 parts by weight or less relative to 100 parts by weight of the biodegradable polymer. The blended amount of the second thermoplastic polymer may be 1 part by weight or more and 1500 parts by weight or less relative to 100 parts by weight of the biodegradable polymer.

Advantageous Effects of Invention

According to the present disclosure, there can be provided porous particles having excellent biodegradability, texture, and soft-focus properties, and a cosmetic composition containing the porous particles.

Brief Description of Drawings

FIG. 1 is a scanning electron microscope (SEM) image (magnification: 3000 times) of porous particles of Example A-1.
FIG. 2 is a scanning electron microscope (SEM) image (magnification: 3000 times) of porous particles of Example A-1.
FIG. 3 is a scanning electron microscope (SEM) image (magnification: 3000 times) of particles of Comparative Example A-2.
FIG. 4 is a scanning electron microscope (SEM) image (magnification: 3000 times) of particles of Comparative Example A-2.

Description of Embodiments

The present disclosure will be described in detail below with reference to preferred embodiments. The configurations, combinations thereof, and the like in each of the embodiments are an example, and various additions, omissions, substitutions, and other changes may be made as appropriate without departing from the spirit of the present disclosure. The present disclosure is not limited by the embodiments and is limited only by the claims. Each aspect disclosed in the present specification can be combined with any other feature disclosed herein.

In the present specification, "X to Y" indicating a range means "X or more and Y or less", "ppm" means "ppm by weight", and unless otherwise noted, all test temperatures are room temperature (20°C $\pm$ 5°C).

Porous particles

The porous particles according to an embodiment of the present disclosure are particles containing a biodegradable polymer as a main component. The porous particles have an average particle size of 0.08 μm or more and 100 μm or less, a sphericity of 0.7 or more and 1.0 or less, and a relative specific surface area of more than 3.0 and 20 or less. The biodegradable polymer is one type or two or more types selected from an aliphatic polyester and a polysaccharide ester. The total degree of substitution of the polysaccharide ester is 0.7 or more and 3.0 or less. Here, the term "porous" means a state in which a large number of pores are formed on the surface and inside of a particle. The pores may be independent pores, continuous pores, or a mixture of both types of pores. The term "main component" means that a component contained in the largest amount among constituent components of the particle is a biodegradable polymer, and the content thereof is at least 50 wt.%.

The porous particles according to an embodiment of the present disclosure are formed from a biodegradable material, and exhibit excellent texture and soft-focus properties due to the shape thereof. Furthermore, the porous particles have high oil absorbability. The porous particles according to an embodiment of the present disclosure can be blended into various cosmetic compositions. By blending the porous particles, it is possible to obtain a high-quality cosmetic composition with less environmental load.

Here, the term "biodegradable polymer" refers to a polymer that degrades in soil or seawater or in a living body. In the present specification, the "polymer" is defined as a compound having a structure formed by repetitive bonding of one constituent unit or two or more constituent units. The polymer may be a synthetic polymer or a naturally occurring polymer as long as the polymer exhibits predetermined biodegradability. The biodegradable polymer according to an embodiment of the present disclosure is an aliphatic polyester and/or a polysaccharide ester having a total degree of substitution of 0.7 or more and 3.0 or less. The porous particles may further contain a biodegradable polymer such as an aliphatic polyol, an aliphatic polycarbonate, or a polyacid anhydride as long as the effects of the present disclosure can be obtained.

The type of the aliphatic polyester is not particularly limited, and examples thereof include a polyhydroxyalkanoate having, as a repeating unit, a constituent unit obtained by polycondensation of a hydroxyalkanoic acid, and a polymer having, as a repeating unit, a constituent unit obtained by dehydration condensation of an aliphatic dicarboxylic acid and an aliphatic diol, from the viewpoint of a polymer structure.

Examples of the polyhydroxyalkanoate include polyglycolic acid, polylactic acid, poly(β-hydroxybutyric acid), poly(β-hydroxyvaleric acid), poly(lactic acid-co-glycolic acid), poly(β-hydroxybutyric acid-co-β-hydroxyvaleric acid), poly(β-propiolactone), and poly(ε-caprolactone). Examples of the polymer of an aliphatic dicarboxylic acid and an aliphatic diol include polyethylene succinate, polybutylene succinate, and poly(butylene succinate-co-butylene adipate).

From the viewpoint of easy availability and excellent biodegradability, a preferred aliphatic polyester is one type or two or more types selected from the group consisting of polycaprolactone, polyhydroxybutyric acid, and polylactic acid. Other aliphatic polyesters not specified herein may be used as long as the effects of the present disclosure are obtained.

From the viewpoint of easily achieving a desired particle shape, the weight average molecular weight of the aliphatic polyester is preferably 10000 or more, more preferably 20000 or more, still more preferably 50000 or more. From the viewpoint of excellent biodegradability, the weight average molecular weight of the aliphatic polyester is preferably 5000000 or less, more preferably 1000000 or less, still more preferably 500000 or less, and particularly preferably 250000 or less. The weight average molecular weight of the aliphatic polyester may be 10000 to 5000000, 10000 to 1000000, 10000 to 500000, 10000 to 250000, 20000 to 5000000, 20000 to 1000000, 20000 to 500000, 20000 to 250000, 50000 to 5000000, 50000 to 1000000, 50000 to 500000, or 50000 to 250000.

The weight average molecular weight of the aliphatic polyester is determined by performing size exclusion chromatography (GPC) measurement using the following apparatus under the following conditions (GPC-light scattering method).

Apparatus: GPC "SYSTEM-21H", available from Shodex

Solvent: acetone
Column: two columns of GMHxl (Tosoh Corporation), guard column (TSKgel guard column HXL-H available from Tosoh Corporation)
Flow rate: 0.8 ml/min
Temperature: 29°C
Sample concentration: 0.25% (wt/vol)
Injection volume: 100 μl
Detection: MALLS (multi-angle light scattering detector) ("DAWN-EOS" available from Wyatt Technology Corporation)
Reference material for MALLS calibration: PMMA (molecular weight: 27600)

The polysaccharide ester is a carboxylic acid ester of a polysaccharide, and is defined as a compound in which some of hydroxyl groups in the molecular chain are substituted with acyl groups. The type of the polysaccharide is not particularly limited as long as the effects of the present disclosure can be obtained, and examples of the polysaccharide include cellulose, starch, chitin, chitosan, and collagen. Two or more polysaccharide esters may be used in combination. Carboxylic acid esters of other polysaccharides not specified herein may be used as long as the effects of the present disclosure are obtained.

The total degree of substitution of the polysaccharide ester used in the porous particles according to an embodiment of the present disclosure is appropriately selected within a range of 0.7 or more and 3.0 or less depending on the type of the polysaccharide and the type of the substituent. The total degree of substitution of the polysaccharide ester may be 1.2 or more, 1.5 or more, or 2.0 or more, and 2.95 or less, 2.80 or less, or 2.65 or less. The total degree of substitution of the polysaccharide ester can be measured by a known method using $^{13}$C-NMR or $^1$H-NMR.

From the viewpoint of easy availability and excellent biodegradability, a preferred polysaccharide ester is a cellulose ester, and a cellulose acylate having an acyl group having 3 or more carbons is more preferred. The number of carbons of the acyl group of the cellulose acylate may be 4 or more, or 5 or more, and 20 or less, or 15 or less. The cellulose acylate may have two or more acyl groups as substituents. The cellulose acylate may have an acetyl group as a substituent together with an acyl group having 3 or more carbons. In the present disclosure, two or more cellulose acylates having different numbers of carbons and degrees of substitution of acyl groups may be used in combination as the biodegradable polymer.

Specific examples of the cellulose acylate in the present disclosure include cellulose propionate, cellulose butyrate, cellulose acetate propionate, and cellulose acetate butyrate.

From the viewpoint of achieving good biodegradability, the total degree of substitution of the cellulose acylate is preferably 2.95 or less, more preferably 2.80 or less, and still more preferably 2.65 or less. From the viewpoint of high melt fluidity, the total degree of substitution of the cellulose acylate is preferably 1.20 or more, more preferably 1.50 or more, and still more preferably 2.10 or more. The total degree of substitution of the cellulose acylate may be 1.20 to 2.95, 1.20 to 2.80, 1.20 to 2.65, 1.50 to 2.95, 1.50 to 2.80, 1.50 to 2.65, 2.10 to 2.95, 2.10 to 2.80, or 2.10 to 2.65.

The degree of substitution of the cellulose acylate can be measured by the following method. For example, the measurement can be performed according to the method described in Tezuka (Tezuka, Carbonydr. Res. 273, 83 (1995)) using NMR. That is, a free hydroxyl group of the cellulose acylate is acylated with a carboxylic anhydride in pyridine. The type of the carboxylic anhydride used here should be selected depending on the purpose of the analysis; for example, when a degree of propionyl substitution of cellulose propionate is analyzed, acetic anhydride is suitably used. The resulting sample is dissolved in deuterated chloroform, and the $^{13}$C-NMR spectrum is measured. When a cellulose acylate having a propionyl group or a cellulose acylate having no propionyl group is treated with propionic anhydride and then the degree of propionyl substitution is analyzed, carbonyl carbon signals of the propionyl group appear in the same order of the 2-, 3-, and 6-positions from a high magnetic field in the region from 172 ppm to 174 ppm. The total degree of substitution of the cellulose acylate treated with the carboxylic anhydride by the method of Tezuka or a similar method is 3.0, and thus, if the sum of areas of the carbonyl carbon signals of the acyl group originally included in the cellulose acylate and the carbonyl signals of the acyl group introduced by the treatment with carboxylic anhydride is normalized to 3.0, and the presence ratio of each acyl group at each corresponding position (in other words, area ratio of each signal) is determined, each of the degrees of acyl substitution at the 2-, 3-, and 6-positions of a glucose ring in the cellulose ester can be obtained. It goes without saying, a substituent containing an acyl group that can be analyzed by this method is only a substituent group that does not correspond to the carboxylic anhydride used in the treatment for an analytical purpose. The degree of substitution can be analyzed by $^1$H-NMR in addition to $^{13}$C-NMR.

The weight average molecular weight of the cellulose acylate is not particularly limited as long as the effects of the present disclosure are obtained. From the viewpoint of easily achieving a desired shape, the weight average molecular weight of the cellulose acylate is preferably 10000 or more, more preferably 20000 or more, still more preferably 30000 or more. From the viewpoint of high biodegradability and achieving melt fluidity, the weight average molecular weight of the cellulose acylate is preferably 500000 or less, more preferably 400000 or less, still more preferably 300000 or less. The weight average molecular weight of the cellulose acylate may be 10000 to 500000, 10000 to 400000, 10000 to 300000, 20000 to 500000, 20000 to 400000, 20000 to 300000, 30000 to 500000, 30000 to 400000, or 30000 to 300000. The weight average molecular weight of the cellulose ester of the cellulose acylate is measured in the same manner as in the aliphatic polyester described above.

While the average particle size of the porous particles according to an embodiment of the present disclosure is 0.08 μm or more and 100 μm or less, the average particle size may be 0.10 μm or more, 1.0 μm or more, 2.0 μm or more, or 4.0 μm or more. The average particle size may be 80 μm or less, 40 μm or less, 20 μm or less, or 14 μm or less. When the average particle size is more than 100 μm, the texture may be impaired. It is difficult to produce porous particles having an average particle size of less than 0.08 μm. The "texture" in the present specification is a concept including skin feeling and tactile sensation in the case of directly touching the porous particles, and, for example, in the case of touching a cosmetic composition containing the porous particles.

The average particle size of the porous particles can be measured using dynamic light scattering. In the present specification, the average particle size ($\mu$m) means the value of the particle size corresponding to 50% of the integrated scattering intensity in this volume-based particle size distribution.

The particle size variation coefficient of the porous particles according to an embodiment of the present disclosure may be 0% or more and 60% or less, or 2% or more and 50% or less.

The particle size variation coefficient of the porous particles is calculated by the following formula.

Particle size variation coefficient (%) = standard deviation of particle size ($\mu$m)/average particle size ($\mu$m) $\times$ 100

Details of the method of measuring the average particle size and the particle size variation coefficient of the porous particles and the measurement conditions will be described below in Examples.

While the sphericity of the porous particles according to an embodiment of the present disclosure is 0.7 or more and 1.0 or less, the sphericity may be 0.8 or more and 1.0 or less, or 0.9 or more and 1.0 or less. Porous particles having a sphericity of less than 0.7 provide poor texture, and for example, a cosmetic composition containing such particles may have a poor skin feeling.

The sphericity of the porous particles is determined as an average value of the minor axis length/major axis length ratios of particles obtained using an image of the particles observed with a scanning electron microscope (SEM). The particles can be determined to be closer to a true sphere as the sphericity approaches 1. Details of the method of measuring the sphericity will be described below in Examples.

The porous particles according to an embodiment of the present disclosure have a relative specific surface area (RSSA) of more than 3.0 and 20 or less. The relative specific surface area is preferably 5.0 or more, more preferably 7.0 or more, still more preferably 8.5 or more, even more preferably 9.0 or more, and particularly preferably 10.0 or more. The relative specific surface area may be 18.0 or less. Particles having a relative specific surface area of 3.0 or less correspond to truly spherical fine particles having a smooth surface and having no or very few pores, and the particles are less likely to deform against an applied external force and are inferior in texture (particularly softness). Particles having a relative specific surface area of more than 20 make it difficult to maintain high sphericity, and are inferior in texture.

The relative specific surface area (RSSA) is defined by the following relational expression.

RSSA = specific surface area measurement value/theoretical specific surface area

The theoretical specific surface area is a specific surface area calculated from the measurement results of particle size distribution on the assumption that the particles are truly spherical particles having a smooth surface, and is defined by the following formula.

$$\text{Theoretical specific surface area} = (1/d)\Sigma(P\imath * S\imath/V\imath)$$

d: True specific gravity (constant at 1350 (kg/m$^3$)) P$\imath$: Distribution (volume fraction) S$\imath$: Surface area of one particle (m$^2$), i.e., surface area of a truly spherical particle having a diameter L$\imath$ (m): $(4/3) * \pi * (L\imath/2)^3$ V$\imath$: Volume (m$^3$) of one particle, i.e., volume of a spherical particle having a diameter L$\imath$ (m): $4\pi * (L\imath/2)^2$

The specific surface area measurement value is determined by a nitrogen BET specific surface area measurement method. Details of specific surface area measurement by the BET method will be described below in Examples.

The degree of surface smoothness of the porous particles according to an embodiment of the present disclosure is preferably 10% or more and 95% or less, more preferably 50% or more and 92% or less, and still more preferably 75% or more and 90% or less. In particles having a degree of surface smoothness of less than 10%, the proportion of portions (pore portions) corresponding to recesses in the particles is very large. Thus, the particles encounter difficulty in having a truly spherical shape. Therefore, a sphericity of 0.7 or more may fail to be achieved, resulting in poor texture. Meanwhile, "the degree of surface smoothness exceeds 95%" means that the proportion of pores in particles is very small, or no pores are present in the particles. Thus, the particles are less likely to deform against an external force applied thereto and are inferior in texture (particularly softness), and a sufficient oil absorption amount may fail to be achieved.

The degree of surface smoothness of the porous particles can be determined as follows: a scanning electron micrograph of the particles is obtained, recesses and protrusions of the particle surfaces are observed, and the degree of surface smoothness is determined based on the area of recessed portions on the surfaces. Details of the method of measuring the degree of surface smoothness will be described in the Examples below.

The bulk specific gravity of the porous particles according to an embodiment of the present disclosure may be 0.1 or more and 0.9 or less, 0.2 or more and 0.9 or less, or 0.2 or more and 0.7 or less. For example, when the particles are blended in a cosmetic composition, the higher the bulk specific gravity of the particles, the better the fluidity of the cosmetic composition. The bulk specific gravity can be measured by a method in accordance with JIS K 1201-1.

The oil absorption amount of the porous particles according to an embodiment of the present disclosure as measured by using linseed oil is preferably 60 ml or more, more preferably 70 ml or more, and still more preferably 80 ml or more per 100 g of the porous particles. The oil absorption amount may be 200 ml or less, and is preferably 100 ml or less, and more preferably 90 ml or less. When the oil absorption amount is 60 ml or more per 100 g of the porous particles, the porous particles have particularly excellent texture. For example, when the porous particles are blended in a cosmetic composition, the cosmetic composition exhibits further improved skin feeling. In the case where the oil absorption amount is more than 200 ml per 100 g of the porous particles, when a cosmetic composition containing the porous particles is used, oil in the skin is absorbed more than necessary by the cosmetic composition, which may cause dryness. From the viewpoint of preventing excessive drying and providing a cosmetic composition having good skin feeling, the oil absorption amount may be 60 ml to 200 ml, 60 ml to 100 ml, 60 ml to 90 ml, 70 ml to 200 ml, 70 ml to 100 ml, 70 ml to 90 ml, 80 ml to 200 ml, 80 ml to 100 ml, or 80 ml to 90 ml per 100 g of the porous particles.

The oil absorption amount as measured by using linseed oil can be determined by JIS K5101-13-1:2004 (ISO 787-5:1980), Test methods for pigments - Part 13: Oil absorption amount - Section 1: Refined linseed oil method.

According to another aspect of the present disclosure, the porous particles may contain a plasticizer. In the present disclosure, the plasticizer refers to a compound which is used for a biodegradable polymer and can increase the plasticity of the biodegradable polymer. The type of the plasticizer is not particularly limited, and examples thereof include polyvalent carboxylic acid esters, for example, adipic acid-based plasticizers, including adipic acid esters such as dimethyl adipate, dibutyl adipate, diisostearyl adipate, diisodecyl adipate, diisononyl adipate, diisobutyl adipate, diisopropyl adipate, diethylhexyl adipate, dioctyl adipate, dioctyldodecyl adipate, dicapril adipate, dihexyldecyl adipate, di(ethylene glycol monoalkyl ether) adipate, di(diethylene glycol monomethyl ether) adipate, di(triethylene glycol monomethyl ether) adipate, di(tetraethylene glycol monomethyl ether) adipate, di(pentaethylene glycol monomethyl ether) adipate, di(hexaethylene glycol monomethyl ether) adipate, di(propylene glycol monoalkyl ether) adipate, di(dipropylene glycol monomethyl ether) adipate, di(tripropylene glycol monomethyl ether) adipate, di(tetrapropylene glycol monomethyl ether) adipate, di(pentapropylene glycol monomethyl ether) adipate, di(hexapropylene glycol monomethyl ether) adipate, di(polyethylene glycol monoalkyl ether) adipate, di(polypropylene glycol monoalkyl ether) adipate, diphenyl adipate, dinaphthyl adipate, and dibenzyl adipate; citric acid-based plasticizers, including citric acid esters such as acetyl triethyl citrate, acetyl tributyl citrate, isodecyl citrate, isopropyl citrate, triethyl citrate, triethylhexyl citrate, and tributyl citrate; glutaric acid-based plasticizers, including glutaric acid esters such as diisobutyl glutarate, dioctyl glutarate, and dimethyl glutarate; succinic acid-based plasticizers, including succinate esters such as diisobutyl succinate, diethyl succinate, diethylhexyl succinate, and dioctyl succinate; sebacic acid-based plasticizers, including sebacic acid esters such as diisoamyl sebacate, diisooctyl sebacate, diisopropyl sebacate, diethyl sebacate, diethylhexyl sebacate, and dioctyl sebacate; and phthalic acid-based plasticizers, including phthalic acid esters such as ethyl phthalate, methyl phthalate, diaryl phthalate, diethyl phthalate, diethylhexyl phthalate, dioctyl phthalate, dibutyl phthalate, and dimethyl phthalate. These polyvalent carboxylic acid esters may be mixed polybasic acid esters.

The plasticizer for the biodegradable polymer to be used may be glycerin-based plasticizers including glycerin alkyl esters such as triacetin, diacetin, and monoacetin; neopentyl glycol; and phosphate-based plasticizers including phosphate esters such as trioleyl phosphate, tristearyl phosphate, and tricetyl phosphate. In addition, di-2-methoxyethyl phthalate, dibutyl tartrate, ethyl O-benzoylbenzoate, ethyl phthalyl ethyl glycolate (EPEG), methyl phthalyl ethyl glycolate (MPEG), N-ethyltoluenesulfonamide, O-cresyl p-toluenesulfonate, triethyl phosphate (TEP), triphenyl phosphate (TPP), and tripropionin may be used. These plasticizers may be used alone, or two or more plasticizers may be used in combination.

From the viewpoint of having a high effect of plasticizing the biodegradable polymer, a polyvalent carboxylic acid-based plasticizer or a glycerin-based plasticizer is preferred, and one type or two or more types selected from mixed polybasic acid esters or glycerin alkyl esters are more preferred. The plasticizer for the biodegradable polymer may be a commercially available plasticizer, such as trade name "DAIFATTY-10" available from Daihachi Chemical Industry Co., Ltd., trade names "BIOCIZER", "RIKEMAL PL-004" and "Poem G-002" available from Riken Vitamin Co., Ltd., and trade names "POLYSIZER" and "MONOSIZER" available from DIC Corporation.

When the porous particles according to an embodiment of the present disclosure contain a plasticizer, the content of the plasticizer contained in the porous particles is not particularly limited. For example, the content of the plasticizer in the porous particles may be more than 0 parts by weight and 120 parts by weight or less, 2 parts by weight or more and 100 parts by weight or less, 10 parts by weight or more and 80 parts by weight or less, or 15 parts by weight or more and 50 parts by weight or less, relative to 100 parts by weight of the biodegradable polymer. The content of the plasticizer in the porous particles can be determined by $^1$H-NMR measurement.

In still another aspect of the present disclosure, a portion or the entirety of the surfaces of the porous particles may be coated with an inorganic powder. Because of the inorganic powder present on the particle surfaces, the porous particles can achieve surface physical properties suitable for a solvent and a preparation used in a cosmetic composition. According to the porous particles having the inorganic powder, high particle dispersibility is achieved in various solvents and preparations, and the texture of the resulting cosmetic composition is improved. The inorganic powder and the porous particles may be physically attached to each other or may be chemically bonded to each other.

The particle shape of the inorganic powder is not particularly limited as long as the effects of the present disclosure are obtained, and the particle shape may be, for example, any of a spherical shape, a plate-like shape, a needle-like shape, a granular shape, and an irregular shape. The average particle size of the inorganic powder is preferably smaller than the average particle size of the porous particles, and may be, for example, 1/3 or less or 1/10 or less of the average particle size of the porous particles. The average particle size of the inorganic powder is a volume-based median size, and is measured in the same manner as in the average particle size of the porous particles.

The type of the inorganic powder is not particularly limited, and examples thereof include titanium oxide, silicon oxide, aluminum oxide, zinc oxide, zirconium oxide, magnesium oxide, boron nitride, silicon nitride, barium sulfate, calcium sulfate, magnesium sulfate, calcium carbonate, magnesium carbonate, talc, mica, kaolin, sericite, mica, vermiculite, hydirite, bentonite, montmorillonite, hectorite, kaolinite, zeolite, ceramic powder, hydroxyapatite, calcium phosphate, silicic acid, aluminum silicate, magnesium silicate, aluminum magnesium silicate, and calcium silicate. Two or more types may be used in combination. One type or two or more types selected from the group consisting of titanium oxide, silicon oxide, aluminum oxide, zinc oxide, and zirconium oxide are preferred from the viewpoint of good adhesion to the porous particles and achieving good texture.

The amount of the inorganic powder added to the porous particles is preferably 1.0 wt.% or more, more preferably 3.0 wt.% or more, and particularly preferably 5.0 wt.% or more from the viewpoint of achieving surface properties suitable for blending into the cosmetic composition. From the viewpoint that the physical properties of the porous particles are not inhibited, the addition amount of the inorganic powder is preferably 50.0 wt.% or less, more preferably 30.0 mass% or less, and particularly preferably 10.0 wt.% or less. When two or more types of inorganic powders are used in combination, the total amount thereof preferably satisfies the above range.

The porous particles according to an embodiment of the present disclosure have excellent biodegradability. For example, the biodegradation rate measured by a method using activated sludge according to JIS K6950 is preferably 40 wt.% or more, more preferably 50 wt.% or more, and still more preferably 60 wt.% or more within 30 days.

Cosmetic composition

The porous particles according to an embodiment of the present disclosure exhibit good texture in addition to excellent biodegradability, and thus can be suitably used for various cosmetic compositions. Since the porous particles according to an embodiment of the present disclosure have high sphericity, when the porous particles are blended into a cosmetic composition, they fill in and smooth out the recesses and protrusions of the skin and scatter light in various directions, and thus provide an effect of making wrinkles less noticeable (soft-focus). Moreover, since the porous particles exhibit a high oil absorption amount, the porous particles blended in the cosmetic composition can absorb sebum and prevent makeup deterioration.

Examples of the cosmetic composition include foundations, such as liquid foundation and powder foundation; concealers; sunscreens; makeup bases; lipsticks and lipstick bases; Oshiroi face powders, such as body powders, solid face powders, and face powders; solid powder eyeshadows; wrinkle masking creams; and skin and hair external preparations mainly for cosmetic purposes, such as skin care lotions. The form of the cosmetic composition is not limited. The cosmetic form may be any of a liquid preparation, such as an aqueous solution, a milky lotion, or a suspension; a semi-solid preparation, such as a gel or a cream; or a solid preparation, such as a powder, a granule, or a solid. In addition, the cosmetic form may be, for example, an emulsion preparation, such as a cream or a milky lotion; an oil gel preparation, such as a lipstick; a powder preparation, such as a foundation; or an aerosol preparation, such as a hair styling agent. The cosmetic composition containing the porous particles according to an embodiment of the present disclosure, particularly the liquid foundation, has excellent spreadability to the skin, covering power for spots and freckles, and slipperiness.

Method for producing porous particles

The porous particles according to an embodiment of the present disclosure can be produced by sequentially performing the following steps (1) to (3):

(1) preparing a mixture by mixing a biodegradable polymer, a plasticizer, a first thermoplastic polymer, and a second thermoplastic polymer different from the first thermoplastic polymer;

(2) melt-kneading the prepared mixture at 200°C or higher and 280°C or lower to yield a kneaded product; and
(3) removing the first thermoplastic polymer and the second thermoplastic polymer from the obtained kneaded product.

The biodegradable polymer used in the production method according to an embodiment of the present disclosure is one type or two or more types selected from aliphatic polyesters and polysaccharide esters. The total degree of substitution of the polysaccharide ester is 0.7 or more and 3.0 or less. The aliphatic polyesters and polysaccharide esters described above for the porous particles are appropriately selected and used. The aliphatic polyesters and polysaccharide esters can be produced by known methods. A commercially available biodegradable polymer may be used as long as the effects of the present disclosure are obtained.

For example, when a cellulose acylate having a total degree of substitution of 0.7 or more and 3.0 or less is used as the polysaccharide ester, the cellulose acylate is prepared through activating a raw material pulp (cellulose); acylating the activated cellulose with an esterifying agent (acylating agent); deactivating the acylating agent after completion of the acylation reaction; and aging (saponifying, hydrolyzing) the produced cellulose acylate to adjust the total degree of substitution to a desired value. The method may include, prior to the activation step, a pretreatment step; i.e., disintegrating and grinding the raw material pulp, and then spraying and mixing acetic acid with the resultant pulp. The method may include a post-treatment step after the aging (saponifying, hydrolyzing) step; i.e., separation by precipitation, purification, stabilization, and drying.

The total degree of substitution of the cellulose acylate can be adjusted by controlling the conditions of aging (conditions such as time and temperature). The type of substituent can be determined by selection of the esterifying agent. The total degree of substitution of the produced cellulose acylate may be 0.7 or more and 3.0 or less, 1.20 or more and 2.95 or less, 1.50 or more and 2.80 or less, or 2.10 or more and 2.65 or less.

The type of the plasticizer used in the production method according to an embodiment of the present disclosure is not particularly limited as long as it has a plasticizing effect in the melt extrusion of the biodegradable polymer. The plasticizer can be appropriately selected depending on the type and physical properties of the biodegradable polymer to be used. Specifically, one of the above-described plasticizers to be contained in the porous particles may be used alone or two or more types thereof may be used in combination. From the viewpoint that the effect of plasticizing the biodegradable polymer is high, a polycarboxylic acid-based plasticizer or a glycerin-based plasticizer is preferred, and one type or two or more types selected from mixed polybasic acid esters or glycerin alkyl esters are more preferred.

The blended amount of the plasticizer may be more than 0 parts by weight and 120 parts by weight or less, 2 parts by weight or more and 100 parts by weight or less, 10 parts by weight or more and 80 parts by weight or less, or 15 parts by weight or more and 50 parts by weight or less, relative to 100 parts by weight of the biodegradable polymer. When the blended amount is excessively small, the sphericity of the resulting porous particles tends to decrease, whereas when the blended amount is excessively large, the shape of the particles cannot be maintained, resulting in a decreasing tendency of the sphericity.

In the present specification, the "thermoplastic polymer" means a polymer having a wide range of thermoplasticity, and usually means a polymer having a weight average molecular weight of 10000 or more. The types of the first thermoplastic polymer and the second thermoplastic polymer are not particularly limited, and those satisfying the following relational expression can be appropriately selected and used depending on the type of the biodegradable polymer.

$$0.05 \le |SPc - SPa|/|SPb - SPa| \le 1.5$$

In the expression, SPa is an SP value of the biodegradable polymer, SPb is the SP value of the first thermoplastic polymer, and SPc is the SP value of the second thermoplastic polymer. |SPc - SPa| represents an absolute value of a difference between SPc and SPa, and |SPb - SPa| represents an absolute value of a difference between SPb and SPa. The SP value is a Hildebrand solubility parameter ($\delta$) and is a physical property value defined by the square root of cohesive energy density.

The first and second thermoplastic polymers are more preferably those satisfying $0.07 \le |SPc - SPa|/|SPb - SPa| \le 1.0$, and still more preferably those satisfying $0.1 \le |SPc - SPa|/|SPb - SPa| \le 1.0$. In the case of $|SPc - SPa|/|SPb - SPa| < 0.05$ or $|SPc - SPa|/|SPb - SPa| > 1.5$, small pores are formed in the produced porous particles, and the number of pores is also small, and thus the relative specific surface area (RSSA) may decrease, resulting in poor texture.

In the production method according to an embodiment of the present disclosure, it is preferable that both the first thermoplastic polymer and the second thermoplastic polymer have water solubility. In other words, first and second water-soluble polymers are preferred. Here, the "water-soluble" polymer means that an insoluble content is less than 50 wt.% when 1 g of the polymer is dissolved in 100 g of water at 25°C.

Examples of the first thermoplastic polymer or the second thermoplastic polymer include polyvinyl alcohol, polyethylene glycol, sodium polyacrylate, polyvinylpyrrolidone, polypropylene oxide, polyglycerin, polyethylene oxide, polyvinyl acetate, modified starch, thermoplastic starch, methyl cellulose, ethyl cellulose, hydroxyethyl cellulose, and hydroxypropyl cellulose. Thermoplastic starch can be prepared by a known method. For example, thermoplastic starch can be produced with reference to JP H06-6307 B or WO 92/04408 by mixing tapioca starch with approximately 20% of glycerin as a plasticizer, and then kneading the mixture with a twin-screw extruder.

The first thermoplastic polymer preferably contains at least one selected from the group consisting of polyvinyl alcohol, sodium polyacrylate, polyvinylpyrrolidone, and thermoplastic starch, and more preferably contains at least one selected from the group consisting of polyvinyl alcohol and thermoplastic starch. The weight average molecular weight of polyvinyl alcohol is preferably 500 or more and 50000 or less.

In the production method according to an embodiment of the present disclosure, a thermoplastic polymer different from the first thermoplastic polymer is selected as the second thermoplastic polymer. When the first thermoplastic polymer is selected from the group consisting of polyvinyl alcohol, sodium polyacrylate, polyvinylpyrrolidone, and thermoplastic starch, the second thermoplastic polymer is preferably polyethylene glycol. The weight average molecular weight of polyethylene glycol used as the second thermoplastic polymer is preferably 500 or more and 50000 or less.

Here, the weight average molecular weight (Mw) is a value obtained by multiplying individual molecules by their molecular weights and taking a weighted average, and is determined by gel permeation chromatography (GPC).

The blended amount of the first thermoplastic polymer is preferably 110 parts by weight or more and 15000 parts by weight or less, more preferably 180 parts by weight or more and 1200 parts by weight or less, still more preferably 200 parts by weight or more and 800 parts by weight or less relative to 100 parts by weight of the biodegradable polymer. When the blended amount is less than 110 parts by weight, non-spherical irregular porous particles having poor sphericity may be produced. When the blended amount exceeds 15000 parts by weight, the resulting porous particles may have a very small particle size.

The blended amount of the second thermoplastic polymer is preferably 1 part by weight or more and 1500 parts by weight or less, more preferably 2 parts by weight or more and 150 parts by weight or less, and still more preferably 3 parts by weight or more and 100 parts by weight or less relative to 100 parts by weight of the biodegradable polymer. When the blended amount is less than 1 part by weight, the resulting porous particles may have an insufficient number of pores formed therein, resulting in an insufficient oil absorption amount.

As long as the effects of the present disclosure are obtained, the biodegradable polymer, the plasticizer, the first thermoplastic polymer, and the second thermoplastic polymer may be mixed in one stage or in multiple stages. The biodegradable polymer, the plasticizer, the first thermoplastic polymer, and the second thermoplastic polymer may be mixed by melt-kneading. For example, the biodegradable polymer may be mixed or melt-kneaded with the plasticizer to prepare a first mixture, and then the first mixture may be blended with the first thermoplastic polymer and the second thermoplastic polymer, followed by mixing or melt-kneading.

The mixing of the biodegradable polymer and the plasticizer, or the mixing of the biodegradable polymer, the plasticizer, the first thermoplastic polymer, and the second thermoplastic polymer can be performed in a dry or wet process using a mixer such as a Henschel mixer. In the case of using a mixer such as a Henschel mixer, the temperature in the mixer is preferably such a temperature that the biodegradable polymer does not melt or degrade, for example, a temperature in a range of 20°C or higher and lower than 200°C.

When the mixing of the biodegradable polymer and the plasticizer or the mixing of the biodegradable polymer, the plasticizer, the first thermoplastic polymer, and the second thermoplastic polymer is performed by melt-kneading, the mixing may be performed at a temperature in a range of 20°C or higher and lower than 200°C using a mixer such as a Henschel mixer, followed by melt-kneading. When the biodegradable polymer and the plasticizer, or the biodegradable polymer, the plasticizer, the first thermoplastic polymer, and the second thermoplastic polymer are more homogeneously mixed together within a short period of time, the finally prepared porous particles have high sphericity and provide improved texture and touch feeling.

The melt-kneading may be carried out by thermal mixing with an extruder. The kneading temperature (cylinder temperature) of the extruder may be in a range of 200°C to 230°C. The melt-kneading performed at a temperature in this range enables plasticization to provide a uniform kneaded product. When the kneading temperature is excessively low, the resulting particles may have lowered sphericity, resulting in poor texture and touch feeling. When the kneading temperature is excessively high, the kneaded product may be denatured or colored by heat. A decrease in the viscosity of the melted product due to a high kneading temperature may lead to insufficient kneading of the resin in a twin-screw extruder.

For example, when cellulose acylate is used as the biodegradable polymer, the kneading temperature (cylinder temperature) of the twin-screw extruder may be 200°C. The kneaded product may be extruded in a strand shape and then formed into a pellet shape by hot cutting or the like. In this case, the die temperature may be approximately 220°C.

In the production method according to an embodiment of the present disclosure, a mixture containing the biodegradable polymer, the plasticizer, the first thermoplastic polymer, and the second thermoplastic polymer is melt-kneaded at 200°C or higher and 280°C or lower to prepare a kneaded product. When the above-described biodegradable polymer, plasticizer, first thermoplastic polymer, and second thermoplastic polymer are mixed by melt-kneading at 200°C or higher and 280°C or lower, the kneaded product prepared by the mixing may be directly subjected to the subsequent step.

An extruder such as a twin-screw extruder can be used for melt-kneading the mixture. When an extruder is used, the kneading temperature means the cylinder temperature. The kneaded product containing the biodegradable polymer may be extruded from a die attached to a tip of the extruder into a string shape and then cut into pellets. In this case, the die temperature may be 220°C or higher and 300°C or lower.

According to the present disclosure, removing the first thermoplastic polymer and the second thermoplastic polymer from the kneaded product containing the biodegradable polymer, the plasticizer, the first thermoplastic polymer, and the second thermoplastic polymer produces porous particles containing the biodegradable polymer as a main component and having an average particle size of 0.08 $\mu$m or more and 100 $\mu$m or less, a sphericity of 0.7 or more and 1.0 or less, and a relative surface area of more than 3.0 and 20 or less.

Examples of the method of removing the first thermoplastic polymer and the second thermoplastic polymer include a method in which the kneaded product is brought into contact with a good solvent of the first thermoplastic polymer and the second thermoplastic polymer, and the first and second thermoplastic polymers are removed by eluting the polymers into the solvent. Examples of the solvent include water; alcohols such as methanol, ethanol, and isopropanol; and mixed solvents thereof. Specifically, the first thermoplastic polymer and the second thermoplastic polymer can be removed from the kneaded product by mixing the kneaded product containing the biodegradable polymer, the plasticizer, the first thermoplastic polymer, and the second thermoplastic polymer with a solvent, eluting the first thermoplastic polymer and the second thermoplastic polymer into the solvent, and then removing a filtered product by filtration.

In the process of removing the first thermoplastic polymer and the second thermoplastic polymer from the kneaded product, the plasticizer may be or may not be removed together with the first thermoplastic polymer and the second thermoplastic polymer. Therefore, the resulting porous particles may or may not contain the plasticizer.

From the viewpoint of high removal efficiency of the first thermoplastic polymer and the second thermoplastic polymer, in mixing proportions of the kneaded product and the solvent, the content of the kneaded product is preferably 0.01 wt.% or more and 20 wt.% or less, more preferably 2 wt.% or more and 15 wt.% or less, and still more preferably 4 wt.% or more and 13 wt.% or less with respect to the total weight of the kneaded product and the solvent. When the content of the kneaded product is more than 20 wt.%, the first thermoplastic polymer and the second thermoplastic polymer may not be sufficiently removed. It may be difficult to separate a solid component containing the porous particles and a liquid component containing the dissolved first and second thermoplastic polymers by an operation such as filtration or centrifugation.

From the viewpoint of high removal efficiency of the first and second thermoplastic polymers, the mixing temperature of the kneaded product and the solvent is preferably 0°C or higher and 200°C or lower, more preferably 20°C or higher and 110°C or lower, still more preferably 40°C or higher and 80°C or lower. When the mixing temperature is lower than 0°C, the first and second thermoplastic polymers may not be sufficiently dissolved and may be difficult to remove. When the mixing temperature exceeds 200°C, it may be difficult to produce particles having a desired shape due to deformation or aggregation of the particles.

The mixing time of the kneaded product and the solvent is not particularly limited, and may be appropriately adjusted. For example, the mixing time may be 0.5 hours or more, 1 hour or more, 3 hours or more, or 5 hours or more, and 6 hours or less.

A stirring device such as an ultrasonic homogenizer or a Three-One Motor can be used for a method of mixing the kneaded product and the solvent to elute the first and second thermoplastic polymers. For example, when a Three-One Motor is used as the stirring device, the rotation speed during mixing of the kneaded product and the solvent may be 5 rpm or more and 3000 rpm or less. Thus, the first and second thermoplastic polymers can be efficiently removed from the kneaded product. The plasticizer can also be efficiently removed from the kneaded product.

In an embodiment of the present disclosure, an inorganic powder is added to and mixed with porous particles produced by removing the first and second thermoplastic polymers from the kneaded product. This process produces porous particles in which a portion or the entirety of the surfaces is coated with the inorganic powder. The porous particles have further improved texture.

The inorganic powder is preferably one type or two or more types selected from the group consisting of titanium oxide, silicon oxide, aluminum oxide, zinc oxide, and zirconium oxide from the viewpoint of achieving good texture. The addition amount of the inorganic powder is preferably 0.01 parts by weight or more and 1.0 part by weight or less relative to 100 parts by weight of the biodegradable polymer.

The method of adding the inorganic powder to and mixing it with the porous particles produced by removing the first and second thermoplastic polymers is not particularly limited, and a known mixing means is appropriately selected and used. The method may involve dry mixing or wet mixing. For example, the dry mixing may involve the use of a mixing device such as a ball mill, a sand mill, a bead mill, a homogenizer, a planetary mixer, or FILMIX. The order of mixing the porous particles and the inorganic powder is not particularly limited. The porous particles and the inorganic powder may be simultaneously charged into the mixing device, or a predetermined amount of the inorganic powder may be charged into the mixing device and stirred (or pulverized simultaneously with stirring), and then the porous particles may be charged and mixed.

If necessary, the production method according to an embodiment of the present disclosure may include a step of drying the produced porous particles after removing the first and second thermoplastic polymers and/or adding and mixing the inorganic powder. The drying method is not particularly limited, and a known method such as heat drying, reduced-pressure drying, or vacuum drying can be used. From the viewpoint of high drying efficiency, the drying temperature is preferably room temperature or higher, and may be 50°C or higher or 60°C or higher. From the viewpoint of suppressing thermal degradation, the drying temperature is preferably 120°C or lower.

Examples

The present disclosure will next be specifically described with reference to Examples, but the technical scope of the present disclosure is not limited by these Examples. The configurations and combinations thereof in each of the embodiments are an example, and various additions, omissions, substitutions, and other changes may be made as appropriate without departing from the spirit of the present disclosure.

Example A-1

100 parts by weight of polyhydroxybutyric acid (PHB, available from Goodfellow, weight average molecular weight 550000, SP value: 21.8 mPa$^{1/2}$) as a biodegradable polymer and 43 parts by weight of a mixed dibasic acid ester (trade name "DAIFATTY-10" available from DAIHACHI CHEMICAL INDUSTRY CO., LTD.) as a plasticizer were dry-blended, dried at 80°C for 12 hours or more, and further stirred and mixed using a Henschel mixer to prepare a mixture of the biodegradable polymer and the plasticizer. The resultant mixture was fed to a twin-screw extruder (PCM30 available from Ikegai Corp., cylinder temperature: 200°C, die temperature: 220°C), melt-kneaded, and extruded to thereby prepare pellets.

The resultant pellets were dry-blended with 310 parts by weight of polyvinyl alcohol (PVA, available from The Nippon Synthetic Chemical Industry Co., Ltd.: melting point: 190°C, degree of saponification: 99.1%, SP value: 34 (mPa$^{1/2}$) as a first thermoplastic polymer and 24 parts by weight of polyethylene glycol (PEG, SP value: 20 mPa$^{1/2}$) as a second thermoplastic polymer, then the mixture was supplied to a twin-screw extruder (PCM30 available from Ikegai Corp., cylinder temperature: 220°C, die temperature: 220°C), and melt-kneaded and extruded to produce a kneaded product containing the aliphatic polyester, the plasticizer, and the first and second thermoplastic polymers.

The produced kneaded product was mixed with pure water (solvent) such that the concentration was equal to or lower than 5 wt.% (= the weight of kneaded product/(the weight of kneaded product + the weight of pure water) × 100), and the mixture was stirred using Three-One Motor (BL-3000 available from Shinto Scientific Co., Ltd.) at a rotational speed of 100 rpm and a temperature of 80°C for 3 hours. The solution after stirring was filtered with filter paper (No. 5A available from ADVANTEC), and the filtered product was removed. The removed filtered product was again mixed with pure water such that the concentration of the kneaded product was adjusted to 5 wt.% or less, and the mixture was further stirred at a temperature of 80°C and a rotation speed of 100 rpm for 3 hours. After filtration, an operation of stirring the filtered product in water was repeated three times or more to produce porous particles of Example A-1.

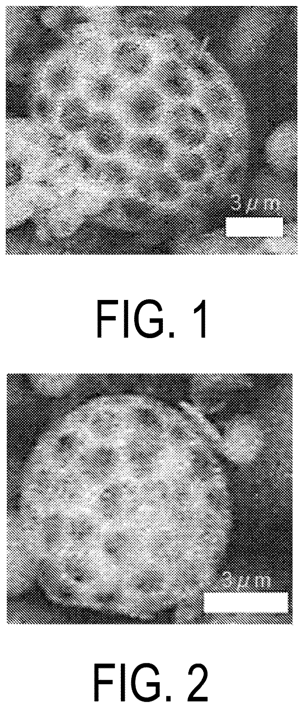

The average particle size (μm), particle size variation coefficient (%), sphericity, degree of surface smoothness (%), and relative specific surface area (RSSA) of the produced porous particles were measured, and the porous particles were evaluated for biodegradability and texture. The evaluation results are shown in Table 1. The measurement or evaluation of the average particle size, the particle size variation coefficient, the sphericity, the degree of surface smoothness, the RSSA, the biodegradability, and the texture were performed by the following methods. Scanning electron microscope (SEM) images of the porous particles of Example A-1 are illustrated in FIG. 1 (magnification 3000: times) and FIG. 2 (magnification 3000: times). The length of a scale bar in FIG. 1 is 3.0 μm.

Average particle size and particle size variation coefficient

The average particle size was measured using dynamic light scattering. Firstly, a sample was mixed with pure water, and the mixture was treated with an ultrasonic vibrator to prepare a suspension having a concentration of about 100 ppm. Subsequently, the volume-frequency particle size distribution was determined by laser diffractometry (using "Laser Diffraction/Scattering Particle Size Distribution Measuring Device LA-960" available from Horiba Ltd., ultrasonic treatment for 15 minutes, refractive index (1.500), medium (water; 1.333)). A value of the particle size corresponding to 50% of the integrated scattering intensity in this volume-frequency particle size distribution was defined as an average particle size (μm). The particle size variation coefficient (%) was calculated using the following formula.

Particle size variation coefficient (%) = standard deviation of particle size (μm)/average particle size (μm) × 100

Sphericity

Using an image of particles observed with a scanning electron microscope (SEM), the major axis lengths and the minor axis lengths of 30 randomly selected particles were measured to determine the (minor axis length)/(major axis length) ratio of each particle, and the average value of the (minor axis length)/(major axis length) ratios was defined as the sphericity.

Degree of surface smoothness

An image of the particles observed with a scanning electron microscope (SEM) was binarized using an image processor Winroof (available from Mitani Corporation). A region including the center and/or a vicinity of the center of one particle was randomly selected from the binarized image. The area ratio of a portion (shade portion) corresponding to recesses of unevenness in the region was calculated, and the degree of surface smoothness (%) of the one particle was calculated by the following formula:

Degree of surface smoothness of one particle (%) = (1 - the area ratio of recesses) x 100 Area ratio of recesses = the area of recesses in the randomly selected region/the area of the randomly selected region

The average value of the degrees of surface smoothness of ten randomly selected particle samples (n1 to n10) was defined as a degree of surface smoothness (%). The larger the value, the higher the degree of surface smoothness of the particles. The region used for calculating the area ratio may be any region smaller than one particle, including the center and/or a vicinity of the center of the one particle. The size of the region may be 5 $\mu$m square when the particle size is 15 $\mu$m.

Relative specific surface area: RSSA

Assuming that the particles were truly spherical fine particles having a smooth surface, the relative specific surface area (RSSA) was calculated by the following formula with the specific surface area calculated from the measurement results of particle size distribution as "theoretical specific surface area" and the specific surface area measured by the BET method as "specific surface area measurement value".

RSSA = specific surface area measurement value/theoretical specific surface area

The "specific surface area measurement value" in the formula was measured using a nitrogen BET specific surface area measurement method. A sample was preliminarily heated and evacuated at a temperature of 100°C for about 1 hour using MasterPrep degassing apparatus available from Quantachrome Instruments, and then the nitrogen adsorption amount was measured at about 7 points in a relative pressure range of 0.05 to 0.28 by a nitrogen gas adsorption method using a specific surface area measuring apparatus ("Autosorb iQ Station 2" available from Quantachrome Instruments), and the specific surface area was calculated by using the BET method.

Biodegradability

The biodegradability was evaluated by biodegradation rate. The biodegradation rate was measured by a method using activated sludge according to JIS K6950. The activated sludge was obtained from an urban sewage treatment plant. A supernatant (activated sludge concentration: about 360 ppm) obtained by allowing the activated sludge to stand for about 1 hour was used in an amount of about 300 mL per culture bottle. The measurement was started at the time point when 30 mg of a sample was stirred in the supernatant, and thereafter, the measurement was performed 31 times in total at intervals of 24 hours until the elapse of 720 hours; i.e., 30 days. Details of the measurement are as follows. A biochemical oxygen demand (BOD) in each culture bottle was measured using a coulometer OM3001 available from Ohkura Electric Co., Ltd. The percentage of the biochemical oxygen demand (BOD) relative to the theoretical biochemical

**EP 4 455 198 A1**

oxygen demand (BOD) in complete degradation based on the chemical composition of each sample was defined as the biodegradation rate (wt.%), and the biodegradability was evaluated according to the following criteria.

◎: more than 60 wt.%
○: 40 wt.% or more and 60 wt.% or less
△: 10 wt.% or more and less than 40 wt.%
✕: less than 10 wt.%

Texture

Sensory evaluation was performed by a panel test of 5 persons for the texture of the particles. The persons were instructed to touch the particles to evaluate comprehensively softness, smoothness, and moist feeling as the texture, on a scale with a maximum score of 5 points according to the following criteria. The average score of the 5 persons was calculated.
Good: 5, Slightly good: 4, Fair: 3, Slightly poor: 2, and Poor: 1.

Scanning electron microscope (SEM) image

A scanning electron microscope (SEM) image was obtained at a magnification of 3000. A scanning electron microscope (trade name "TM 3000") available from Hitachi High-Technologies Corporation was used for taking an image.

Examples A-2 to A-27 and Comparative Examples A-1 to A-6

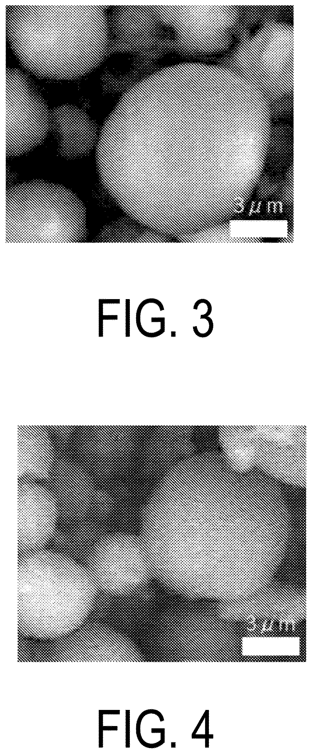

Porous particles of Examples A-2 to A-27 and Comparative Examples A-2, A-4, and A-6 were produced in the same manner as in Example A-1 except that the types and blended amounts of the biodegradable polymer, the plasticizer, the first thermoplastic polymer, and the second thermoplastic polymer were changed as shown in Table 1-4. In Comparative Examples A-1, A-3, and A-5, no plasticizer was added, and thus the melt-kneading of the biodegradable polymer was insufficient, and fine particles failed to be formed. The physical properties of the produced porous particles were evaluated by the method described above. The evaluation results are shown in Table 1-4. Scanning electron microscope (SEM) images of the porous particles of Comparative Example A-2 are illustrated in FIG. 3 (magnification: 3000 times) and FIG. 4 (magnification: 3000 times).

[Table 1]

**14**

(Table 1)

| | Example A-1 | Example A-2 | Example A-3 | Example A-4 | Example A-5 | Example A-6 | Example A-7 | Example A-8 | Example A-9 |
|---|---|---|---|---|---|---|---|---|---|
| Biodegradable polymer | PHB | PHB | PHB | PHB | PHB | PHB | PHB | PHB | PHB |
| Blended amount (part(s) by weight) | 100 | 100 | 100 | 100 | 100 | 100 | 100 | 100 | 100 |
| Plasticizer | DAIFATTY-101 | DAIFATTY-101 | DAIFATTY-101 | DAIFATTY-101 | DAIFATTY-101 | DAIFATTY-101 | DAIFATTY-101 | DAIFATTY-101 | DAIFATTY-101 |
| Blended amount (part(s) by weight) | 43 | 3 | 11 | 11 | 11 | 11 | 25 | 100 | 100 |
| First thermoplastic polymer | PVA | PVA | PVA | PVA | PVA | PVA | PVA | PVA | PVA |
| Blended amount (part(s) by weight) | 310 | 223 | 241 | 248 | 222 | 944 | 271 | 433 | 1700 |
| Second thermoplastic polymer | PEG | PEG | PEG | PEG | PEG | PEG | PEG | PEG | PEG |
| Blended amount (part(s) by weight) | 24 | 17 | 19 | 11 | 37 | 56 | 21 | 33 | 100 |
| Average particle size ($\mu$m) | 6.1 | 7.2 | 5.6 | 5.8 | 4.4 | 2.9 | 4.8 | 4.4 | 4.1 |
| Particle size variation coefficient (%) | 42 | 45 | 43 | 45 | 42 | 40 | 43 | 41 | 40 |
| Sphericity | 0.8 | 0.8 | 0.7 | 0.7 | 0.7 | 0.8 | 0.7 | 0.7 | 0.7 |
| Degree of surface smoothness (%) | 71 | 80 | 75 | 70 | 75 | 80 | 72 | 70 | 75 |
| Theoretical specific surface area ($m^2$/g) | 0.51 | 0.42 | 0.51 | 0.48 | 0.58 | 0.92 | 0.65 | 0.62 | 0.64 |
| BET specific surface area ($m^2$/g) | 5.81 | 3.78 | 3.98 | 4.80 | 4.58 | 10.30 | 6.05 | 5.08 | 6.53 |
| RSSA | 11.4 | 9.0 | 7.8 | 10.0 | 7.9 | 11.2 | 9.3 | 8.2 | 10.2 |
| Biodegradability | ◎ | ◎ | ◎ | ◎ | ◎ | ◎ | ◎ | ◎ | ◎ |

(continued)

| | Example A-1 | Example A-2 | Example A-3 | Example A-4 | Example A-5 | Example A-6 | Example A-7 | Example A-8 | Example A-9 |
|---|---|---|---|---|---|---|---|---|---|
| Texture | 4.2 | 4.9 | 4.7 | 4.5 | 4.4 | 4.5 | 4.3 | 4.1 | 4.2 |

[Table 2]

(Table 2)

| | Example A-10 | Example A-11 | Example A-12 | Example A-13 | Example A-14 | Example A-15 | Example A-16 | Example A-17 | Example A-18 |
|---|---|---|---|---|---|---|---|---|---|
| Biodegradable polymer | PCL | PCL | PCL | PCL | PCL | PCL | PCL | PCL | PCL |
| Blended amount (part(s) by weight) | 100 | 100 | 100 | 100 | 100 | 100 | 100 | 100 | 100 |
| Plasticizer | DAIFATTY-101 | DAIFATTY-101 | DAIFATTY-101 | DAIFATTY-101 | DAIFATTY-101 | DAIFATTY-101 | DAIFATTY-101 | DAIFATTY-101 | D AIF ATTY-101 |
| Blended amount (part(s) by weight) | 3 | 11 | 11 | 11 | 11 | 25 | 43 | 100 | 100 |
| First thermoplastic polymer | PVA | PVA | PVA | PVA | PVA | PVA | PVA | PVA | PVA |
| Blended amount (part(s) by weight) | 223 | 241 | 248 | 222 | 944 | 271 | 310 | 433 | 1700 |
| Second thermoplastic polymer | PEG | PEG | PEG | PEG | PEG | PEG | PEG | PEG | PEG |
| Blended amount (part(s) by weight) | 17 | 19 | 11 | 37 | 56 | 21 | 24 | 33 | 100 |
| Average particle size ($\mu$m) | 6.2 | 4.2 | 4.7 | 3.2 | 1.8 | 3.6 | 3.3 | 3.4 | 2.9 |
| Particle size variation coefficient (%) | 45 | 43 | 45 | 42 | 40 | 43 | 42 | 41 | 40 |
| Sphericity | 0.8 | 0.7 | 0.7 | 0.7 | 0.8 | 0.7 | 0.8 | 0.7 | 0.7 |
| Degree of surface smoothness (%) | 80 | 75 | 70 | 75 | 80 | 72 | 71 | 70 | 75 |
| Theoretical specific surface area ($m^2$/g) | 0.48 | 0.57 | 0.58 | 0.62 | 0.81 | 0.67 | 0.45 | 0.57 | 0.49 |
| BET specific surface area ($m^2$/g) | 4.27 | 4.96 | 5.22 | 8.18 | 9.80 | 4.82 | 4.73 | 6.44 | 5.29 |
| RSSA | 8.9 | 8.7 | 9 | 13.2 | 12.1 | 7.2 | 10.5 | 11.3 | 10.8 |

(continued)

| | Example A-10 | Example A-11 | Example A-12 | Example A-13 | Example A-14 | Example A-15 | Example A-16 | Example A-17 | Example A-18 |
|---|---|---|---|---|---|---|---|---|---|
| Biodegradability | ◎ | ◎ | ◎ | ◎ | ◎ | ◎ | ◎ | ◎ | ◎ |
| Texture | 4.6 | 4.2 | 4.5 | 4.1 | 4.2 | 4.4 | 4.2 | 4.1 | 4.2 |

[Table 3]

(Table 3)

| | Example A-19 | Example A-20 | Example A-21 | Example A-22 | Example A-23 | Example A-24 | Example A-25 | Example A-26 | Example A-27 |
|---|---|---|---|---|---|---|---|---|---|
| Biodegradable polymer | CAB | CAB | CAB | CAB | CAB | CAB | CAB | CAB | CAB |
| Blended amount (part(s) by weight) | 100 | 100 | 100 | 100 | 100 | 100 | 100 | 100 | 100 |
| Plasticizer | Triacetin | Triacetin | Triacetin | Triacetin | Triacetin | Triacetin | Triacetin | Triacetin | Triacetin |
| Blended amount (part(s) by weight) | 3 | 11 | 11 | 11 | 11 | 25 | 43 | 100 | 100 |
| First thermoplastic polymer | PVA | PVA | PVA | PVA | PVA | PVA | PVA | PVA | PVA |
| Blended amount (part(s) by weight) | 223 | 241 | 248 | 222 | 944 | 271 | 310 | 433 | 1700 |
| Second thermoplastic polymer | PEG | PEG | PEG | PEG | PEG | PEG | PEG | PEG | PEG |
| Blended amount (part(s) by weight) | 17 | 19 | 11 | 37 | 56 | 21 | 24 | 33 | 100 |
| Average particle size ($\mu$m) | 7.8 | 6.2 | 5.7 | 4.2 | 2.8 | 4.6 | 4.3 | 2.4 | 1.9 |
| Particle size variation coefficient (%) | 45 | 43 | 45 | 42 | 40 | 43 | 42 | 41 | 40 |
| Sphericity | 0.8 | 0.7 | 0.7 | 0.7 | 0.8 | 0.7 | 0.8 | 0.7 | 0.7 |
| Degree of surface smoothness (%) | 80 | 75 | 70 | 75 | 80 | 72 | 71 | 70 | 75 |
| Theoretical specific surface area ($m^2$/g) | 0.83 | 0.78 | 0.67 | 0.77 | 0.34 | 0.82 | 0.91 | 0.45 | 0.51 |
| BET specific surface area ($m^2$/g) | 6.47 | 5.62 | 5.56 | 5.78 | 3.23 | 6.89 | 6.92 | 4.77 | 4.13 |
| RSSA | 7.80 | 7.20 | 8.30 | 7.50 | 9.50 | 8.40 | 7.60 | 10.60 | 8.10 |
| Biodegradability | ○ | ○ | ○ | ○ | ○ | ○ | ○ | ○ | ○ |
| Texture | 4.9 | 4.8 | 4.3 | 4.2 | 4.3 | 4.3 | 4.1 | 4.3 | 4.1 |

[Table 4]

(Table 4)

| | Comparative Example A-1 | Comparative Example A-2 | Comparative Example A-3 | Comparative Example A-4 | Comparative Example A-5 | Comparative Example A-6 |
|---|---|---|---|---|---|---|
| Biodegradable polymer | PHB | PHB | PCL | PCL | CAB | CAB |
| Blended amount (part(s) by weight) | 100 | 100 | 100 | 100 | 100 | 100 |
| Plasticizer | DAIFATTY-101 | DAIFATTY-101 | DAIFATTY-101 | DAIFATTY-101 | Triacetin | Triacetin |
| Blended amount (part(s) by weight) | 0 | 18 | 0 | 11 | 0 | 11 |
| First thermoplastic resin | PVA | PVA | PVA | PVA | PVA | PVA |
| Blended amount (part(s) by weight) | 217 | 275 | 217 | 259 | 217 | 259 |
| Second thermoplastic resin | PEG | PEG | PEG | PEG | PEG | PEG |
| Blended amount (part(s) by weight) | 17 | 0 parts | 17 | 0 | 17 | 0 |
| Average particle size ($\mu$m) | - | 5.7 | - | 2.8 | - | 3.5 |
| Particle size variation coefficient (%) | - | 38 | - | 40 | - | 42 |
| Sphericity | - | 0.8 | - | 0.9 | - | 0.8 |
| Degree of surface smoothness (%) | - | 98 | - | 100 | - | 99 |
| Theoretical specific surface area ($m^2$/g) | - | 0.68 | - | 0.73 | - | 0.85 |
| BET specific surface area ($m^2$/g) | - | 1.97 | - | 1.83 | - | 2.55 |
| RSSA | - | 2.90 | - | 2.50 | - | 3.00 |
| Biodegradability | - | ◎ | - | ◎ | - | ○ |
| Texture | - | 3.2 | - | 3.3 | - | 3.4 |

The details of the compounds shown in Tables 1 to 4 are as follows.

PHB: polyhydroxybutyric acid (weight average molecular weight: 550000, SP value: 21.8 mPa$^{1/2}$) available from Goodfellow

PCL: polycaprolactone (weight average molecular weight: 50000, SP value: 21.7 mPa$^{1/2}$) available from Daicel Corporation

CAB: Cellulose acetate butyrate (degree of acetyl substitution: 0.9, degree of butyryl substitution: 1.8, weight average molecular weight 70000, SP value: 23.7 mPa$^{1/2}$) available from Sigma-Aldrich Co. LLC

DAIFATTY-10: mixed dibasic acid ester available from DAIHACHI CHEMICAL INDUSTRY CO., LTD.

Triacetin: available from Daicel Corporation

PVA: polyvinyl alcohol (melting point: 190°C, degree of saponification: 99.1%, SP value: 34 mPa$^{1/2}$) available from The Nippon Synthetic Chemical Industry Co., Ltd.

PEG: polyethylene glycol (weight average molecular weight: 35000, SP value: 20 mPa$^{1/2}$) available from Sigma-Aldrich Co. LLC

As shown in Tables 1 to 4, the porous particles of the Examples all have excellent biodegradability and excellent texture, particularly soft texture, as compared with the particles of the Comparative Examples.

Example B-1

Preparation of liquid foundation

Components shown in Table 5 were mixed, and then stirred well, and the mixture was charged into a container to prepare a liquid foundation. The texture of the resulting liquid foundation was evaluated by the method described below. The results are shown in Table 13.

[Table 5]

| (Table 5) Example B-1 | | |
|---|---|---|
| Component | Product name, etc. | wt.% |
| Cyclopentasiloxane | KF-995 (Shin-Etsu Chemical Co., Ltd.) | 15.2 |
| Mineral oil | HICALL K-230 (KANEDA Co., Ltd.) | 5.0 |
| Ethylhexyl methoxycinnamate | Uvinul MC80 (BASF) | 4.0 |
| Isononyl isononanoate | KAK-99 (Kokyu Alcohol Kogyo Co., Ltd.) | 3.0 |
| Disteardimonium hectorite, cyclopentasiloxane, etc. | Bentone Gel VS-5 PC V HV (Elementis) | 3.0 |
| Phytosteryl macadamiate | Plandool-MAS (Nippon Fine Chemical Co., Ltd.) | 0.3 |
| Trimethylsiloxysilicate, polypropylsilsesquioxane | MQ-1640 Flake Resin (Dow Corning Toray Co., Ltd.) | 0.3 |
| PEG-10 dimethicone | KF-6017P (Shin-Etsu Chemical Co., Ltd.) | 1.5 |
| Polyglyceryl oleate-2, polyhydroxystearic acid, polyglyceryl stearate-2 | PolyAquol OS2 (innovacos) | 1.0 |
| Titanium oxide, cyclopentasiloxane, etc. | SDL-Ti70 (Daito Kasei Kogyo Co., Ltd.) | 12.3 |
| Iron oxide, cyclopentasiloxane, etc. | SDL-IOY50 (Daito Kasei Kogyo Co., Ltd.) | 3.0 |
| | SDL-IOR50 (Daito Kasei Kogyo Co., Ltd.) | |
| | SDL-IOB50 (Daito Kasei Kogyo Co., Ltd.) | |
| Example A-1: PHB particles | | 3.0 |
| BG | 1,3-BG (UK) (Daicel Corporation) | 6.0 |
| Phenoxyethanol | Phenoxyethanol-SP (Yokkaichi Chemical Co., Ltd.) | 0.3 |
| Sodium chloride | | 1.0 |
| EDTA-2Na | | 0.03 |

(continued)

| (Table 5) Example B-1 | |
|---|---|
| Purified water | Balance |
| Total | 100.0 |

Texture

Sensory evaluation was performed by a panel test of 5 persons for the texture of the compositions prepared by blending the particles. The persons were instructed to use the compositions to evaluate comprehensively both smoothness and moist feeling, on a scale with a maximum score of 5 points according to the following criteria. The average score of the 5 persons was calculated.

Good: 5, Slightly good: 4, Fair: 3, Slightly poor: 2, and Poor: 1.

Example B-2

Preparation of sunscreen

Components shown in Table 6 were mixed, and then stirred well, and the mixture was charged into a container to prepare a sunscreen. The texture of the resulting sunscreen was evaluated by the method described above. The results are shown in Table 13.

[Table 6]

| (Table 6) Example B-2 | | |
|---|---|---|
| Component | Product name, etc. | wt.% |
| Diethylaminohydroxybenzoyl hexyl benzoate | Uvinul A Plus Glanular (BASF) | 2.00 |
| Bisethylhexyloxyphenol methoxyphenyl triazine | Tnosorb S (BASF) | 0.50 |
| Ethylhexyl methoxycinnamate, BHT | Uvinul MC80 (BASF) | 7.00 |
| Diisopropyl sebacate | IPSE (Nippon Fine Chemical Co., Ltd.) | 10.00 |
| Dimethicone | KF-96A-10CS (Shin-Etsu Chemical Co., Ltd.) | 2.00 |
| Isododecane | Marukasol R (Maruzen Petrochemical Co., Ltd.) | 26.47 |
| Trimethylsiloxysilicate | MQ-1640 Flake Resin (Dow Corning Toray Co., Ltd.) | 1.00 |
| PEG-9 polydimethyl siloxyethyl dimethicone | KF-6028 (Shin-Etsu Chemical Co., Ltd.) | 2.00 |
| Titanium oxide, etc. | DIS-OP-10A (Sakai Chemical Industry Co., Ltd.) | 4.00 |
| Zinc oxide, etc. | DIF-OP-3W (Sakai Chemical Industry Co., Ltd.) | 10.00 |
| Example A-1: PHB particles | | 5.00 |
| Purified water | | 19.30 |
| BG | 1,3-BG (UK) (Daicel Corporation) | 3.00 |
| Phenoxyethanol | Phenoxyethanol-SP (Yokkaichi Chemical Co., Ltd.) | 0.20 |
| Ethanol | | 7.00 |
| Sodium chloride | | 0.50 |
| EDTA-2Na | | 0.03 |
| Total | | 100.0 |

Example B-3

Preparation of powder foundation

After component A shown in Table 7 was roughly mixed, uniformly dissolved component B was added thereto, and the resultant mixture was stirred well. Then, the mixture was charged into a container to prepare a powder foundation. The texture of the resulting powder foundation was evaluated by the method described above. The results are shown in Table 13.

[Table 7]

| (Table 7) Example B-3 | |
|---|---|
| Component | wt.% |
| (Component A) | |
| Example A-1: PHB particles | 7.50 |
| SI01-2 Talc JA-46R | 29.67 |
| Mica Y-2300 | 20.00 |
| SI01-2 Sericite FSE | 33.00 |
| SI01-2 Titanium oxide CR-50 | 6.50 |
| SI-2 Yellow iron oxide LLXLO | 2.30 |
| SI-2 Red iron oxide RED R-516L | 0.59 |
| SI-2 Black iron oxide BL-100 | 0.44 |
| Component (A) total | 100.00 |
| (Component B) | |
| Dimethicone (20) | 20.00 |
| Dimethicone (350) | 20.00 |
| Glyceryl isostearate | 7.20 |
| Triethylhexanoin | 17.00 |
| Octyldodecyl oleate | 31.55 |
| Sorbitan stearate | 1.00 |
| Polyglyceryl-2 oleate | 3.10 |
| Propylparaben | 0.10 |
| Tocopherol | 0.05 |
| Component (B) total | 100.0 |
| (Final blending) | |
| Component A | 90.00 |
| Component B | 100.00 |

Example B-4

Preparation of makeup base

Component C shown in Table 8 was dispersed in component A, and the mixture was stirred well. Thereafter, component B was added thereto, and the resultant mixture was stirred and charged into a container to prepare a makeup base. The texture of the resulting makeup base was evaluated by the method described above. The results are shown in Table 13.

[Table 8]

| (Table 8) Example B-4 | |
|---|---|
| Component | wt.% |
| (Component A) | |
| (Dimethicone/(PEG-10/15)) crosspolymer, dimethicone | 3.50 |
| PEG-9 polydimethylsiloxyethyl dimethicone | 2.00 |
| Dimethicone | 5.00 |
| Isononyl isononanoate | 4.50 |
| Octyl methoxycinnamate | 10.00 |
| Quaternium-18 hectorite | 1.20 |
| (Dimethicone/vinyl dimethicone) crosspolymer, dimethicone | 5.00 |
| Cyclomethicone | 25.00 |
| (Component B) | |
| Purified water | Balance |
| 1,3-Butylene glycol | 5.00 |
| Sodium citrate | 0.20 |
| Preservative | 0.30 |
| (Component C) | |
| Example A-1: PHB particles | 10.00 |
| Total | 100.0 |

Example B-5

Preparation of lipstick base

Component B shown in Table 9 was heated to 60°C, and mixed well. Component C was added thereto and dispersed well, and then component A was further added thereto and dissolved using a microwave oven, followed by mixing well. Thereafter, the mixture was dissolved again by heating using a microwave oven, poured into a mold, and solidified under cooling. The solidified product was put in a lipstick container to prepare a lipstick base. The texture of the resulting lipstick base was evaluated by the method described above. The results are shown in Table 13.

[Table 9]

| (Table 9) Example B-5 | |
|---|---|
| Component | wt.% |
| (Component A) | |
| Ceresin | 4.27 |
| Microcrystalline wax | 1.55 |
| Deresinated candelilla wax | 5.03 |
| High boiling point paraffin | 3.07 |
| (Component B) | |
| Diisostearyl malate | 1.95 |
| Dipentaerythritol fatty acid ester | 6.22 |
| Adsorption refined lanolin | 2.52 |

(continued)

| (Component B) | |
|---|---|
| Liquid lanolin acetate | 13.34 |
| Glyceryl tri-2-ethylhexanoate | 19.02 |
| Liquid paraffin | 7.28 |
| Isotridecyl isononanoate | 3.21 |
| Diglyceryl triisostearate | 4.01 |
| Methylphenyl polysiloxane | 2.41 |
| Para-hydroxybenzoate | 0.07 |
| Diisostearyl malate | Balance |
| Natural type vitamin E | 0.05 |
| (Component C) | |
| Example A-1: PHB particles | 10.00 |
| Total | 100.00 |

Example B-6

Preparation of body powder

Component A shown in Table 10 was mixed well using a mixer. The resulting powder was charged into a container to prepare a body powder. The texture of the resulting body powder was evaluated by the method described above. The results are shown in Table 13.

[Table 10]

| (Table 10) Example B-6 | |
|---|---|
| Component | wt.% |
| (Component A) | |
| Talc | Balance |
| Example A-1: PHB particles | 10.00 |
| Fragrance | Suitable amount |
| Total | 100.00 |

Example B-7

Preparation of solid face powder

A solid face powder is prepared according to a common cosmetic production method. Specifically, talc and a color pigment shown in Table 11 were mixed with a blender. The porous particles (PHB particles) of Example A-1 and all powder components including the color pigment and the talc preliminarily mixed with the blender were stirred using a Henschel mixer. Thereafter, an oil component (binder) was added thereto, and the mixture was heated to 70°C and further stirred, and then subjected to a pulverization process as necessary. The resultant product was compression-molded in a gold dish container to prepare a solid face powder. The texture of the resulting solid face powder was evaluated by the method described above. The results are shown in Table 13.

[Table 11]

| (Table 11) Example B-7 | |
|---|---|
| Component | wt.% |
| (Powder) | |
| Talc | 30.00 |
| Sericite | 20.00 |
| Kaolin | 15.00 |
| Titanium dioxide | 5.00 |
| Zinc myristate | 5.00 |
| Magnesium carbonate | 5.00 |
| Color pigment | Suitable amount |
| Example A-1: PHB particles | 15.00 |
| (Binder) | |
| Tragacanth gum | 3.00 |
| Liquid paraffin | 2.00 |
| Others: a preservative, an antioxidant, and a fragrance are added in suitable amounts as necessary. | |
| Total | 100.00 |

Example B-8

Preparation of solid powder eyeshadow

After powders shown in Table 12 were mixed well, a binder was uniformly dissolved and added to a powder portion, and the resultant mixture was further mixed. Thereafter, a solid powder eyeshadow was prepared by compression molding. The texture of the resulting solid powder eyeshadow was evaluated by the method described above. The results are shown in Table 13.

[Table 12]

| (Table 12) Example B-8 | |
|---|---|
| Component | wt.% |
| (Powder) | |
| Mica | 15.00 |
| Sericite | 5.00 |
| Pigment | 15.00 |
| Pearl pigment | 10.00 |
| Example A-1: PHB particles | 51.00 |
| (Binder) | |
| Methylpolysiloxane | 2.00 |
| (Others) | |
| Sorbitan sesquioleate | 2.00 |
| Others: an antioxidant, a fragrance, and a preservative are added in a suitable amount as necessary. | |
| Total | 100.00 |

Example B-9

A liquid foundation was prepared in the same manner as in Example B-1 except that the porous particles (PHB particles) in Example A-1 in Table 5 were changed to the porous particles (PCL particles) in Example A-10. The texture of the resulting liquid foundation was evaluated by the method described above. The results are shown in Table 13.

Example B-10

A sunscreen was prepared in the same manner as in Example B-2 except that the porous particles (PHB particles) in Example A-1 in Table 6 were changed to the porous particles (PCL particles) in Example A-10. The texture of the resulting sunscreen was evaluated by the method described above. The results are shown in Table 13.

Example B-11

A powder foundation was prepared in the same manner as in Example B-3 except that the porous particles (PHB particles) of Example A-1 in Table 7 were changed to the porous particles (PCL particles) of Example A-10. The texture of the resulting powder foundation was evaluated by the method described above. The results are shown in Table 13.

Example B-12

A makeup base was prepared in the same manner as in Example B-4 except that the porous particles (PHB particles) of Example A-1 in Table 8 were changed to the porous particles (PCL particles) of Example A-10. The texture of the resulting makeup base was evaluated by the method described above. The results are shown in Table 13.

Example B-13

A liquid foundation was prepared in the same manner as in Example B-1 except that cyclopentasiloxane in Table 5 was changed to a mixture obtained by mixing dodecane (PARAFOL 12-97 (Sasol)) and Cetiol Ultimate (undecane:tridecane = 65 wt.%:35 wt.%, available from BASF SE) in equal weights, isononyl isononanoate was changed to a mixture obtained by mixing coco-caprylate (Cetiol C5 (available from BASF SE)), coco-caprylate/caprate (Cetiol C5C (available from BASF SE)), and dicaprylyl carbonate (Cetiol CC (available from BASF SE)) in equal weights, and macadamia nut fatty acid phytosteryl was changed to camellia oil (pure camellia oil (available from Nikko Rica Corporation)). The texture of the resulting liquid foundation was evaluated by the method described above. The results are shown in Table 13.

Example B-14

A sunscreen was prepared in the same manner as in Example B-2 except that isododecane in Table 6 was changed to a mixture obtained by mixing dodecane (PARAFOL 12-97 (Sasol)) and Cetiol Ultimate (undecane:tridecane = 65 wt.%:35 wt.%, available from BASF SE) in equal weights, and diisopropyl sebacate was changed to a mixture obtained by mixing coco-caprylate (Cetiol C5 (available from BASF SE)), coco-caprylate/caprate (Cetiol C5C (available from BASF SE)), and dicaprylyl carbonate (Cetiol CC (available from BASF SE)) in equal weights. The texture of the resulting sunscreen was evaluated by the method described above. The results are shown in Table 13.

Example B-15

A powder foundation was prepared in the same manner as in Example B-3 except that dimethicone in Table 7 was changed to a mixture obtained by mixing dodecane (PARAFOL 12-97 (Sasol)) and Cetiol Ultimate (undecane:tridecane = 65 wt.%:35 wt.%, available from BASF SE) in equal weights, and octyldodecyloleate was changed to a mixture obtained by mixing coco-caprylate (Cetiol C5 (available from BASF SE)), coco-caprylate/caprate (Cetiol C5C (available from BASF SE)), and dicaprylyl carbonate (Cetiol CC (available from BASF SE)) in equal weights. The texture of the resulting powder foundation was evaluated by the method described above. The results are shown in Table 13.

Example B-16

A makeup base was prepared in the same manner as in Example B-4 except that cyclomethicone in Table 8 was changed to a mixture obtained by mixing dodecane (PARAFOL 12-97 (Sasol)) and Cetiol Ultimate (undecane:tridecane = 65 wt.%:35 wt.%, available from BASF SE) in equal weights, and isononyl isononanoate was changed to a mixture obtained by mixing coco-caprylate (Cetiol C5 (available from BASF SE)), coco-caprylate/caprate (Cetiol C5C (available from BASF SE)), and dicaprylyl carbonate (Cetiol CC (available from BASF SE)) in equal weights. The texture of the resulting makeup base was evaluated by the method described above. The results are shown in Table 13.

Example B-17

A powder foundation was prepared in the same manner as in Example B-3 except that mica Y-2300X in Table 7 was changed to a mixture obtained by mixing mica (mica Y-2300X (available from YAMAGUCHI MICA CO., LTD.)), synthetic mica (PDM-10L (available from TOPY INDUSTRIES LIMITED.)), and (fluoride/hydroxide/oxide)/(Mg/K/silicon) (Micromica MK-200K (available from Katakura & Co-op Agri Corporation) in equal weights, sericite was changed to a mixture obtained by mixing barium sulfate (plate-shaped barium sulfate H (available from Sakai Chemical Industry Co., Ltd.)) and boron nitride (SHP-6 (available from Mizushima Ferroalloy Co., Ltd.)) in equal weights, and talc was changed to a mixture obtained by mixing cellulose (NP Fiber W-06MG (available from Nippon Paper Industries Co., Ltd.)) and silica (Godd Ball E-16C (available from SUZUKIYUSHI INDUSTRIAL CO., LTD.)) in equal weights. The texture of the resulting powder foundation was evaluated by the method described above. The results are shown in Table 13.

Example B-18

A body powder was prepared in the same manner as in Example B-6 except that talc in Table 10 was changed to a mixture obtained by mixing cellulose (NP fiber W-06MG (available from Nippon Paper Industries Co., Ltd.)) and silica (Godd Ball E-16C (available from SUZUKIYUSHI INDUSTRIAL CO., LTD.)) of the same weight. The texture of the resulting body powder was evaluated by the method described above. The results are shown in Table 13.

Example B-19

A solid powder eyeshadow was prepared in the same manner as in Example B-8 except that mica (mica Y-2300X (available from YAMAGUCHI MICA CO., LTD.)) in Table 12 was changed to a mixture obtained by mixing mica (mica Y-2300X (available from YAMAGUCHI MICA CO., LTD.)), synthetic mica (PDM-10L (available from TOPY INDUSTRIES LIMITED.)), and (fluoride/hydroxide/oxide)/(Mg/K/silicon) (Micromica MK-200K (available from Katakura & Co-op Agri Corporation) in equal weights, and sericite was changed to a mixture obtained by mixing barium sulfate (plate-shaped barium sulfate H (available from Sakai Chemical Industry Co., Ltd.)) and boron nitride (SHP-6 (available from Mizushima Ferroalloy Co., Ltd.)) in equal weights. The texture of the resulting solid powder eyeshadow was evaluated by the method described above. The results are shown in Table 13.

Example B-20

A liquid foundation was prepared in the same manner as in Example B-1 except that BG in Table 5 was changed to a mixture obtained by mixing glycerin and pentylene glycol (Diol PD (available from Kokyu Alcohol Kogyo Co., Ltd.)) in equal weights. The texture of the resulting liquid foundation was evaluated by the method described above. The results are shown in Table 13.

Example B-21

A sunscreen was prepared in the same manner as in Example B-2 except that BG in Table 6 was changed to a mixture obtained by mixing glycerin and pentylene glycol (Diol PD (available from Kokyu Alcohol Kogyo Co., Ltd.)) in equal weights. The texture of the resulting sunscreen was evaluated by the method described above. The results are shown in Table 13.

Example B-22

A makeup base was prepared in the same manner as in Example B-4 except that 1.3-butylene glycol in Table 8 was changed to a mixture obtained by mixing glycerin and pentylene glycol (Diol PD (available from Kokyu Alcohol Kogyo Co., Ltd.)) in equal weights. The texture of the resulting makeup base was evaluated by the method described above. The results are shown in Table 13.

Comparative Examples B-1 to B-8

In Comparative Examples B-1 to B-8, a liquid foundation, a sunscreen, a powder foundation, a makeup base, a lipstick base, a body powder, a solid face powder, and a solid powder eyeshadow were prepared in the same manner as in Examples B-1 to B-8, respectively, except that the porous particles (PHB particles) of Example A-1 in Tables 5 to 12 were changed to the porous particles (PHB particles) of Comparative Example A-2. The texture of each of the products was evaluated by the method described above. The results are shown in Table 14.

[Table 13]

(Table 13)

|  | Example B-1 | Example B-2 | Example B-3 | Example B-4 | Example B-5 | Example B-6 | Example B-7 | Example B-8 |
|---|---|---|---|---|---|---|---|---|
| Composition | Liquid foundation | Sunscreen | Powder foundation | Makeup base | Lipstick base | Body powder | Solid face powder | Solid powder eyeshadow |
| Particles | Example A-1 | Example A-1 | Example A-1 | Example A-1 | Example A-1 | Example A-1 | Example A-1 | Example A-1 |
| Texture | 4.5 | 4.6 | 4.3 | 4.2 | 4.1 | 4.5 | 4.7 | 4.6 |
|  | Example B-9 | Example B-10 | Example B-11 | Example B-12 | Example B-13 | Example B-14 | Example B-15 | Example B-16 |
| Composition | Liquid foundation | Sunscreen | Powder foundation | Makeup base | Liquid foundation | Sunscreen | Powder foundation | Makeup base |
| Particles | Example A-10 | Example A-10 | Example A-10 | Example A-10 | Example A-1 | Example A-1 | Example A-1 | Example A-1 |
| Texture | 4.1 | 4.2 | 4.1 | 4.3 | 4.2 | 4.3 | 4.2 | 4.1 |
|  | Example B-17 | Example B-18 | Example B-19 | Example B-20 | Example B-21 | Example B-22 |  |  |
| Composition | Powder foundation | Body powder | Solid powder eyeshadow | Liquid foundation | Sunscreen | Makeup base |  |  |
| Particles | Example A-1 | Example A-1 | Example A-1 | Example A-1 | Example A-1 | Example A-1 |  |  |
| Texture | 4.2 | 4.4 | 4.2 | 4.3 | 4.3 | 4.3 |  |  |

[Table 14]

(Table 14)

| | Comparative Example B-1 | Comparative Example B-2 | Comparative Example B-3 | Comparative Example B-4 | Comparative Example B-5 | Comparative Example B-6 | Comparative Example B-7 | Comparative Example B-8 |
|---|---|---|---|---|---|---|---|---|
| Composition | Liquid foundation | Sunscreen | Powder foundation | Makeup base | Lipstick base | Body powder | Solid face powder | Solid powder eyeshadow |
| Particles | Comparative Example A-2 | Comparative Example A-2 | Comparative Example A-2 | Comparative Example A-2 | Comparative Example A-2 | Comparative Example A-2 | Comparative Example A-2 | Comparative Example A-2 |
| Texture | 3.1 | 2.9 | 3.2 | 3.1 | 3.3 | 3.2 | 3.2 | 3.1 |

As shown in Tables 13 and 14, all the cosmetic compositions of Examples B-1 to B-22 containing the porous particles according to an embodiment of the present disclosure had an excellent texture of 4.0 or more, and particularly exhibited soft texture. Since each of the cosmetic compositions contains the porous particles containing a biodegradable polymer as a main component, the cosmetic composition can be expected to have excellent biodegradability.

**Claims**

1. Porous particles comprising a biodegradable polymer as a main component, wherein

   the biodegradable polymer is an aliphatic polyester and/or a polysaccharide ester having a total degree of substitution of 0.7 or more and 3.0 or less; and
   the porous particles have an average particle size of 0.08 $\mu$m or more and 100 $\mu$m or less, a sphericity of 0.7 or more and 1.0 or less, and a relative specific surface area of more than 3.0 and 20 or less.

2. The porous particles according to claim 1, wherein the porous particles have a degree of surface smoothness of 10% or more and 95% or less.

3. The porous particles according to claim 1 or 2, further comprising a plasticizer for the biodegradable polymer.

4. The porous particles according to any one of claims 1 to 3, wherein the plasticizer is selected from the group consisting of a glycerin-based plasticizer and a polycarboxylic acid ester-based plasticizer.

5. The porous particles according to any one of claims 1 to 4, wherein the aliphatic polyester is a polyhydroxyalkanoate or a polymer of an aliphatic dicarboxylic acid and an aliphatic diol.

6. The porous particles according to any one of claims 1 to 5, wherein the aliphatic polyester is one type or two or more types selected from the group consisting of polycaprolactone, polyhydroxybutyric acid, and polylactic acid.

7. The porous particles according to any one of claims 1 to 6, wherein the polysaccharide ester is a cellulose acylate having an acyl group having 3 or more carbons.

8. A cosmetic composition comprising the porous particles described in any one of claims 1 to 7.

9. A method for producing the porous particles described in any one of claims 1 to 7, the method comprising:

   preparing a mixture by mixing a biodegradable polymer, a plasticizer, a first thermoplastic polymer, and a second thermoplastic polymer different from the first thermoplastic polymer;
   melt-kneading the mixture at 200°C or higher and 280°C or lower to yield a kneaded product; and
   removing the first thermoplastic polymer and the second thermoplastic polymer from the kneaded product.

10. The method for producing the porous particles according to claim 9, wherein when an SP value of the biodegradable polymer is SPa, an SP value of the first thermoplastic polymer is SPb, and an SP value of the second thermoplastic polymer is SPc, SPa, SPb, and SPc satisfy the following relational expression:

$$0.05 \leq |SPc - SPa|/|SPb - SPa| \leq 1.5$$

11. The method for producing the porous particles according to claim 9 or 10, wherein the first thermoplastic polymer is selected from the group consisting of polyvinyl alcohol and thermoplastic starch.

12. The method for producing the porous particles according to any one of claims 9 to 11, wherein the second thermoplastic polymer is polyethylene glycol.

13. The method for producing the porous particles according to any one of claims 9 to 12, wherein the plasticizer is selected from the group consisting of a glycerin-based plasticizer and a polycarboxylic acid ester-based plasticizer.

14. The method for producing the porous particles according to any one of claims 9 to 13, wherein a blended amount of the plasticizer is more than 0 parts by weight and 120 parts by weight or less relative to 100 parts by weight of the biodegradable polymer.

15. The method for producing the porous particles according to any one of claims 9 to 14, wherein a blended amount of the first thermoplastic polymer is 110 parts by weight or more and 15000 parts by weight or less relative to 100 parts by weight of the biodegradable polymer, and a blended amount of the second thermoplastic polymer is 1 part by weight or more and 1500 parts by weight or less relative to 100 parts by weight of the biodegradable polymer.

# FIG. 1

# FIG. 2

FIG. 3

FIG. 4

## INTERNATIONAL SEARCH REPORT

International application No.

**PCT/JP2022/041764**

### A. CLASSIFICATION OF SUBJECT MATTER

*C08J 9/26*(2006.01)i; *A61K 8/02*(2006.01)i; *A61K 8/73*(2006.01)i; *A61K 8/85*(2006.01)i; *A61Q 1/00*(2006.01)i; *C08J 3/12*(2006.01)i; *C08K 5/103*(2006.01)i; *C08L 1/10*(2006.01)i; *C08L 1/12*(2006.01)i; *C08L 3/02*(2006.01)i; *C08L 29/04*(2006.01)i; *C08L 67/00*(2006.01)i; *C08L 67/04*(2006.01)i

FI: C08J9/26 102; A61K8/02; A61K8/85; A61K8/73; A61Q1/00; C08L67/00; C08K5/103; C08L1/10; C08L1/12; C08L67/04; C08L29/04 D; C08L3/02; C08J3/12 Z CFD

According to International Patent Classification (IPC) or to both national classification and IPC

### B. FIELDS SEARCHED

Minimum documentation searched (classification system followed by classification symbols)

C08J9/00-9/42; C08J3/00-3/28; A61K8/00-8/99; A61Q1/00-90/00; C08K5/103; C08L1/10; C08L1/12; C08L3/02; C08L29/04; C08L67/00; C08L67/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2020-152851 A (DAICEL CORPORATION) 24 September 2020 (2020-09-24) claims 1, 9-11, 13, paragraphs [0059], [0060], [0062]-[0087] | 1-15 |
| A | WO 2020/188698 A1 (DAICEL CORP.) 24 September 2020 (2020-09-24) claims 1, 4-5, paragraphs [0001], [0133]-[0157] | 1-15 |
| A | JP 6543920 B2 (TORAY INDUSTRIES, INC.) 17 July 2019 (2019-07-17) claim 1, paragraphs [0001], [0032], [0044]-[0056] | 1-15 |
| A | WO 2017/056908 A1 (SEKISUI PLASTICS CO., LTD.) 06 April 2017 (2017-04-06) claims 1-2, paragraphs [0002], [0021], examples | 1-15 |
| A | JP 2020-530865 A (SAMYANG BIOPHARMACEUTICALS CORP.) 29 October 2020 (2020-10-29) claims 1-2, 12, paragraphs [0001], [0029], examples | 1-15 |

☑ Further documents are listed in the continuation of Box C.    ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 December 2022** | **17 January 2023** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**

International application No.

**PCT/JP2022/041764**

**C. DOCUMENTS CONSIDERED TO BE RELEVANT**

| Category* | Citation of document, with indication, where appropriate, of the relevant passages | Relevant to claim No. |
|---|---|---|
| A | JP 2020-026480 A (JGC CATALYSTS & CHEMICALS LTD.) 20 February 2020 (2020-02-20)<br>claim 1, paragraphs [0001], [0015], [0024], examples | 1-15 |

Form PCT/ISA/210 (second sheet) (January 2015)

### INTERNATIONAL SEARCH REPORT
#### Information on patent family members

| International application No. |
| --- |
| **PCT/JP2022/041764** |

| Patent document cited in search report | | | Publication date (day/month/year) | Patent family member(s) | Publication date (day/month/year) |
|---|---|---|---|---|---|
| JP | 2020-152851 | A | 24 September 2020 | US 2020/0299488 A1<br>claims 1, 9-11, 13, paragraphs [0062], [0063], [0066]-[0090] | |
| WO | 2020/188698 | A1 | 24 September 2020 | US 2022/0142900 A1<br>claims 1, 4-5, paragraphs [0001], [0135]-[0159] | |
| JP | 6543920 | B2 | 17 July 2019 | (Family: none) | |
| WO | 2017/056908 | A1 | 06 April 2017 | US 2018/0265663 A1<br>claims 12-13, paragraphs [0002]-[0046], examples | |
| JP | 2020-530865 | A | 29 October 2020 | US 2020/0207930 A1<br>claims 1-2, 12, paragraphs [0002], [0053], examples | |
| JP | 2020-026480 | A | 20 February 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6543920 B
- JP H066307 B
- WO 9204408 A

**Non-patent literature cited in the description**

- **TEZUKA.** *Carbonydr. Res.,* 1995, vol. 273, 83